# EUROPEAN PATENT APPLICATION

(11) **EP 2 631 891 A1**
(43) Date of publication of application: **28.08.2013**
(21) Application number: 11833895.3
(22) Date of filing: 14.10.2011
(51) Int. Cl.: G09B 23/28, A61B 3/00

(54) **METHOD AND SYSTEM FOR SIMULATING/EMULATING VISION VIA INTRAOCULAR DEVICES OR LENSES PRIOR TO SURGERY**

(30) Priority: 20.10.2010 ES 201031540
(71) Applicant: Luque, Sergio Oscar, 08228 Barcelona (ES)
(72) Inventor: PUJOL RAMO, Jaume, E-08232 Barcelona (ES)
(74) Representative: Isern-Jara, Nuria
(86) International application number: PCT/ES2011/070710
(87) International publication number: WO 2012/052585

(57) **Abstract**

A method and system for simulating/emulating vision using intraocular lenses or devices prior to surgery preferably using multifocal and monofocal intraocular lenses. The invention allows the patient, prior to surgery, to compare the differences in the perception of an image as seen with the intraocular lens and with normal vision, determining which type of lens the patient prefers, and in turn allows the ophthalmologist to measure which provides the better visual performance. It comprises a system containing the intraocular lens, called an artificial eye, a lens for transporting the image from the intraocular lens and eliminating the blur introduced by the artificial eye, and at least one telescope or optometer which transports the intermediate image from the intraocular lens onto the patient's pupil. In this manner, the patient is shown how he/she would see after surgery with each type of lens proposed by the ophthalmologist.

## Description

Method and system for simulating/emulating vision using intraocular lenses or devices prior to surgery.

### Object of the invention

The present invention relates to the field of visual optics, ophthalmology and optometry and is applied to the pre-implant simulation-emulation of any type of intraocular device or lens (IOL), and also to the evaluation of the patient's vision once the chosen type of IOL has been implanted.

The method and system object of the invention makes it possible to use any type of IOL on the market, used mainly in the lenses indicated to resolve presbyopia.

This system would make it possible to show the patient how he/she would be able to see after surgery with each type of lens they would like to try, which would be suggested by the ophthalmologist. In addition, visual tasks may be performed as a measure of visual acuity and to evaluate both far vision and near vision contrast sensitivity. The patient would provide feedback on which lens is most to their liking and, in turn, the doctor could provide a qualitative measure on which of the lenses has better optical performance based on the visual tasks deemed appropriate to perform. The patient's crystalline lens must show an acceptable degree of transparency for the psychophysical measures to realistically represent post-surgical conditions.

### Background of the Invention

In ophthalmological medicine, cataract refers to the total or partial opacification of crystalline lens, which leads to diminished vision. Since 1990, surgery known as phacoemulsification has been performed, consisting of the destruction of the patient's clouded crystalline lens and fitting of an intraocular lens (IOL) in the cavity that is formed on removing it, in order to compensate for the loss of refractive power of the eye.

In the vast majority of cases a monofocal IOL is implanted, i.e. a lens with unique power that will make it possible for the patient to see clearly at a distance, which does not exempt the individual from wearing glasses for near vision. This pathology, cataract, is an age-associated disease and it is estimated that 70% of people older than 70 years of age undergo this type of surgery.

People who have finished their working lives and who lead an active life have led to a great deal of pressure on the available supply in this area. This resulted in the emergence of lenses that attempted to remedy presbyopia while simultaneously offering the possibility of seeing objects at a distance and near, thereby avoiding the need to use glasses of any kind. To this end, there are different types of multifocal and bifocal intraocular lenses in the current state of the art, with refractive and diffractive lenses being the most widely used nowadays.

These multifocal lenses, which are quite costly, do not guarantee patient satisfaction, as the perception of images with this type of lens is not the sharpest available. These lenses give the wearer the opportunity to read at the expense of reducing the quality of far vision, although the proportion of such reduced vision is subjective and cannot be experienced by the patient prior to surgery.

Methods or systems are known in the current state of the art that simulate vision through certain devices, such as the instrument for simulating multifocal ophthalmic corrections described in the patent application PCT2/ES2010/070218, or the laser ray-tracing method for quality control of lenses and optical systems in general described in patent ES 2156558, and even in the publication by C. Cánovas, P. M. Prieto, S. Manzanera, A. Mira, P. Artal, "Hybrid adaptive-optics visual simulator", Opt. Lett., 35, 196-198 (2010).

Nonetheless, they all refer to systems that are aimed at manufacturing a customised lens for the patient on the basis of the results obtained, simulating vision using certain devices but none do so with a lens that is available on the market and identical to that which will be subsequently implanted, to enable the patient to see what their vision would be like once they have been operated on.

### Description of the Invention

The method and system for simulating/emulating vision via intraocular lenses or devices prior to surgery object of the present registration, resolves the aforementioned drawbacks, further providing other additional advantages that will be evident from the description provided hereinafter.

The system consists of an artificial eye (3) where the IOL (4) will be housed, whose image is to be assessed. The power of intraocular lenses covers a certain range to compensate for the impairment produced on removing the crystalline lens. This, coupled with the corneal power, delivers an eye whose optical power is approximately about 60 diopters. A lens (7) is subsequently fitted whose functions are to transport the image of the lens to an intermediate plane (8), and, in turn, to eliminate the spherical component produced by the artificial eye (3). This wavefront thus obtained is transported once again through a telescope with unitary magnification, a Badal system (10) for spherical refractive error correction, towards the patient's pupil. In this manner, the image of the lens can be transported virtually on the patient's pupil (16) and therefore the latter would be able to see as if he/she had the lens actually implanted in his/her crystalline lens.

The patient will perform different refractive vision correction tests, i.e. using the glasses the individual uses on a daily basis for farsightedness. In this manner, visual conditions very similar to those the patient will display once the implant has been fitted can be simulated. The crystalline lens, the organ which is removed when the intraocular lens is implanted, should not show any major alterations such as an extensive loss of transparency. In other words, the pre and post surgical results differ noticeably since the crystalline lens that produces significant deterioration of the image in preoperative conditions is removed by surgery.

By moving one or more mirrors or filters (2 and 11), the media through which the patient sees can be exchanged. On the one hand, this provides the patient with a point of reference between vision with and without the lens in question and, on the other hand, the doctor can compare the results obtained from visual tasks. If the results are similar, the patient can be expected to be very satisfied with the lens once it has been implanted. To avoid changes in the size of the image perceived by the patient, the distance (B) between the mirror (2) and the patient's eye (16) has to be very similar to the distance (A) between this mirror (2) and the artificial eye (3). By means of moving or rotating the mirrors (2 and 11), the light from the objects can be deflected to the artificial eye or passed directly to the patient's eye, making it possible to immediately compare the perception of the normal image and the potential lens to be implanted. An alternative system would be to fit an artificial eye without an intraocular lens such that the patient could experience how images are perceived with and without lens by exchanging artificial eyes.

Eliminating the blurring introduced by the artificial eye requires very precise positioning between the artificial eye and the lens which eliminates that blur (7). Therefore, the system has a complementary device that enables this adjustment or calibration to be made. Because this task requires a certain amount of time, each device has a certain number of artificial eyes depending on the number of lenses that are to be tested in one session. This can be implemented using a lens chamber consisting of a wheel that houses different types of intraocular lens. In this manner, by rotating the wheel or chamber different types of lenses may be exchanged.

As expected, the perception of the images of distant objects is diminished when simulation is performed with bifocal, multifocal or progressive lenses. The advantage of these lenses is the ability to see near objects relatively sharply; a condition that is very impaired in presbyopia. Therefore, using this device will provide the patient with an opportunity to assess both the advantages and disadvantages of each particular IOL.

The ophthalmologist, optometrist or auxiliary technician may perform psychophysical tests to anticipate the post-surgical results provided no complications occur during surgery or providing the lens is not incorrectly positioned in the eye in which it has been implanted.

Finally, the patient is provided with a tool to help him/her make the decision on which type of lens he/she would prefer to be implanted based on likes, preferences and daily tasks. Currently, this is done by means of a questionnaire and different tests that provide the ophthalmologist with information that is used to determine, using subjective and ill-defined criteria, which lens the patient will be more satisfied with.

To complete the description that is going to be given hereinafter and in order to help to better understand its characteristics, accompanying the descriptive memory herein are a set of drawings in whose figures, by way of illustration and not by way of limitation, the most significant details of the invention are represented.

### Brief description of the designs

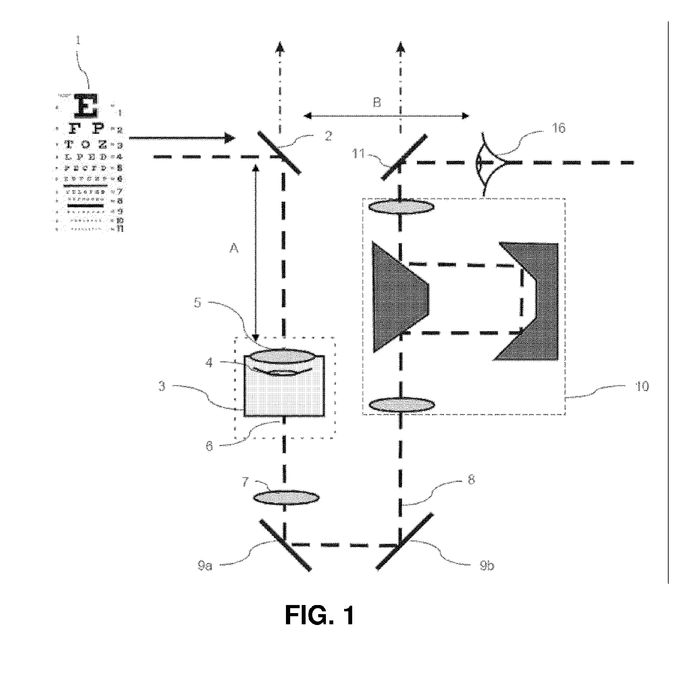

Figure 1.- Diagram of the system for simulation and psychophysical measurement of vision through intraocular lenses prior to surgery according to the present invention.
Figure 2.- System for aligning the patient's eye, placed over the diagram in figure 1.

### Description of a preferred embodiment

In view of the figures discussed and in accordance with the numbering adopted, a preferred although non-limiting embodiment of the invention can be seen, which consists of a system for the simulation and psychophysical measurement of vision using intraocular lenses prior to surgery, in which the light coming from any object, such as for example, an eye chart (1) for measuring visual acuity, is deflected by the mirror (2) toward the artificial eye (3). This artificial eye has a cavity (4) wherein the intraocular lens is inserted. A lens (5) of approximately 40 diopters is fitted at one end, and a window (6) to allow the light to pass through is fitted at the other end. The IOL that is located between these optical components is immersed in water, a saline solution or any liquid that resembles the aqueous humor, so that the artificial eye must be watertight.

This artificial eye (3) forms the image of the object at a distance of about 15 to 30 mm from the intraocular lens, depending on its power. To transport this image to the patient's retina, the 60 diopter lens (7) that performs two functions is positioned, such that on the one hand the image from the eye charts is sent to a state of infinity focus and transports the image of the intraocular lens to a plane (8) after being reflected by a pair of mirrors (9) that introduce a change of direction in the light path. Finally, a Badal system (10) composed of two lenses and two mirrors transports the image formed in the plane (8) over the patient's pupil without the image from the eye charts being modified. An artificial pupil (17) of variable diameter can be positioned over this plane. Although not essential for proper functioning of the system, the lens in question could be assessed at different pupil sizes. The focal plane can be modified by changing the position of the mirrors in the Badal system (10). In this manner, the patient's near-sightedness or far-sightedness can be corrected.

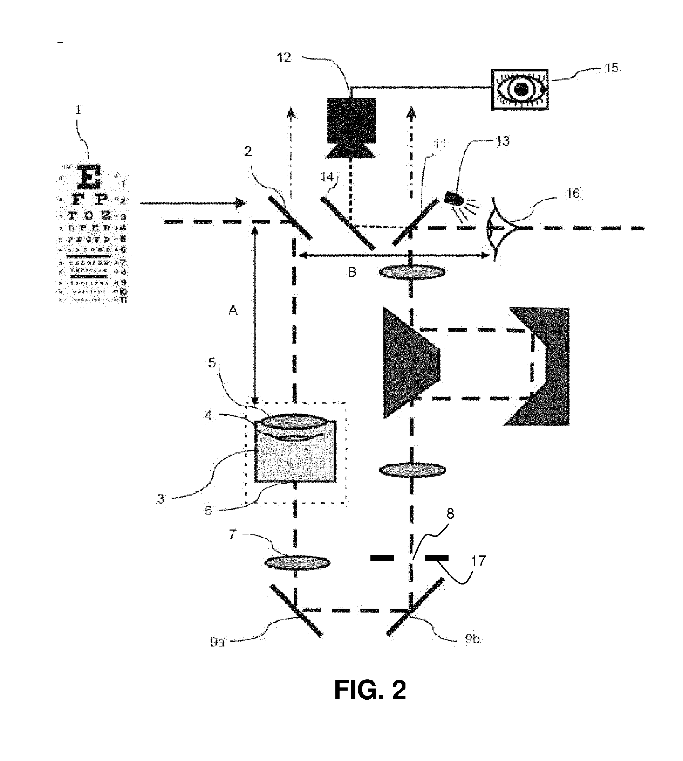

Finally, a high-pass filter (11) deflects the light toward the eye (16). This filter is aligned with the mirror (2) such that when this mirror and the filter (11) are moved from the path of light originating from the eye charts they pass directly to the patient's eye (16), passing through the low-pass filter (14). This allows the user to perform tests and the patient can quickly experience the differences between current vision (displaced mirrors) and the vision the patient will have when the lenses are implanted (mirrors in the position shown in Fig.2).

The simulation can be performed both for distance and reading vision. To avoid any changes in the size of the image when the object is perceived through the intraocular lens or when observing the aforementioned object directly, distance A measured from the centre of the mirror (2) to the artificial eye (3) must be equal to distance B measured from the centre of the mirror (2) to the patient's eye (16). The camera (12) records the image of the eye which is illuminated with infrared light by means of the lighting system (13). This light from the eye is transmitted by the filter (11) and reflected by the filter (14) toward the camera. Finally, the video signal is sent directly to a display (15) or, alternatively, to a computer for display. This is the image the operator has as a reference to align the system.

The details and other accessory elements may be conveniently replaced by others that are technically equivalent and do not depart from the essential nature of the invention or from the scope defined by the claims provided hereinafter.

## Claims

1. A method and system for simulating/emulating vision using intraocular lenses or devices prior to surgery, preferably using multifocal and monofocal intraocular lenses **characterised in that** it allows the patient, prior to surgery, to compare the differences in the perception of an image as seen with the intraocular lens and with normal vision, thereby determining which type of lens the patient prefers, and in turn allows the ophthalmologist to measure which one provides the best visual performance.

2. A method and system for simulating/emulating vision using intraocular lenses or devices prior to surgery according to claim 1, **characterised in that** it comprises a system containing the intraocular lens (3), called an artificial eye, a lens for transporting the image from the intraocular lens and eliminating the blur introduced by the artificial eye, and comprising at least one telescope or optometer that transports the intermediate image from the intraocular lens onto the patient's pupil.

3. A method and system for simulating/emulating vision using intraocular lenses or devices prior to surgery according to claim 1, **characterised in that** the intermediate image of the intraocular lens on the patient's pupil (16) can be transported without modifying the blur or by modifying it in cases of near-sightedness or far-sightedness, enabling the patient, when located in the correct plane, to see real objects through the IOL under conditions similar to those that will exist once the lens is implanted.

4. A method and system for simulating/emulating vision using intraocular lenses or devices prior to surgery according to claim 1, **characterised in that** the telescope or optometer for correcting possible blurring caused by visual defects has two lenses and two mirrors (2,9,11) that can be moved manually or by a motor.

5. A method and system for simulating/emulating vision using intraocular lenses or devices prior to surgery according to claim 2, **characterised in that** the artificial eye (3) has a lens (5) and a window that forms a watertight compartment (4) where the intraocular lens is enclosed, immersed in an aqueous medium, preferably physiological saline.

6. A method and system for simulating/emulating vision using intraocular lenses or devices prior to surgery according to claim 5, **characterised by** the fact that several artificial eyes may optionally be mounted on a rotary drum mechanism enabling the exchange of the IOL that is being simulated, either manually or by a motor.

7. A method and system for simulating/emulating vision using intraocular lenses or devices prior to surgery according to claim 1, **characterised by** the fact that it has a camera (12), a display (15), a filter (14) and a lighting system (13) which makes it possible to view the patient's pupil for correct alignment.

8. A method and system for simulating/emulating vision using intraocular lenses or devices prior to surgery according to claim 1, **characterised by** the fact that it has a mirror (2.11) and a high-pass filter (14) which when moved to a position outside of the path of light coming from the object enables the patient to see the aforementioned object without altering the image in any way, thereby making it possible to compare the differences in the perception of an image as seen with the intraocular lens and with normal vision.

9. A method and system for simulating/emulating vision using intraocular lenses or devices prior to surgery according to claim 1, **characterised in** fact it has a pupil (17) of variable diameter making it possible to assess vision at different pupil sizes.
